# EUROPEAN PATENT SPECIFICATION

(11) **EP 0 931 160 B1**
(45) Date of publication and mention of the grant of the patent: **17.12.2008**
(21) Application number: 96931558.9
(22) Date of filing: 13.09.1996
(51) Int. Cl.: C12P 19/34, G01N 33/543, C12Q 1/68

(54) **MUTATION DETECTION USING PEPTIDE TAGGED IN VITRO SYNTHESIZED PROTEINS**
NACHWEIS VON MUTATIONEN MITTELS IN VITRO SYNTHETISIERTEN PROTEINEN MIT PEPTID-MARKIERUNG
DETECTION DE MUTATIONS A L'AIDE DE PROTEINES SYNTHETISEES IN VITRO MARQUEES A L'AIDE DE PEPTIDES

(43) Date of publication of application: 28.07.1999
(73) Proprietor: Garvin, Alex M., 68480 Durmenach (FR)
(72) Inventor: Garvin, Alex M., 68480 Durmenach (FR)
(74) Representative: Kirkham, Nicholas Andrew
(86) International application number: PCT/US1996/014708
(87) International publication number: WO 1998/011249

(56) References cited:
- US-A- 6 110 738
- US-B1- 6 306 628
- THE JOURNAL OF CELL BIOLOGY. FEB 1995, vol. 128, no. 3, February 1995 (1995-02), pages 363-371, XP002291546 ISSN: 0021-9525
- ROWAN A J ET AL: "INTRODUCTION OF A MYC REPORTER TAG TO IMPROVE THE QUALITY OF MUTATION DETECTION USING THE PROTEIN TRUNCATION TEST" HUMAN MUTATION, WILEY-LISS, NEW YORK, NY, US, vol. 9, no. 2, 1997, pages 172-176, XP009006084 ISSN: 1059-7794
- NATURE GENETICS, June 1995, Vol. 10, HOGERVORST et al., "Rapid Detection of BRCA1 Mutations by the Protein Truncation Test", pages 208-212.
- NEW ENGLAND JOURNAL OF MEDICINE, 30 December 1993, Vol. 329, No. 27, POWELL et al., "Molecular Diagnosis of Familial Adenomatous Polyposis", pages 1982-1987.

## Description

### Background- Field of Invention

This invention relates to a process for detecting mutations in genes by synthesizing the proteins encoded by the genes *in-vitro* with a peptide tag attached to the amino termini, and analyzing properties of the tagged proteins.

### Background- Description of Prior Art

Many genes that predispose to human disease have now been identified and hundreds more will be found over the next decade. In order to take advantage of these findings, the biomedical community must be able to identify individuals that carry mutations in these genes in order to counsel them and to help them take preventative measures.

The methods now being used to screen for mutations in certain disease causing genes suffer from short comings that will make it far too time consuming and expensive to carry out large scale screenings. For example, the breast cancer susceptibility genes BRCA1 (1) and BRCA2 (2) have recently been identified and mutations predisposing to breast cancer have been found throughout the 16 kilobases of coding sequence contained in these two genes. The entire coding sequence of BRCA1 and BRCA2 are now routinely screened by either DNA sequencing (3), Single Strand Conformation Polymorphism (SSCP) (3), or the Protein Truncation Test (PTT) (4). All three of these techniques rely on the electrophoretic properties of either DNA or protein, and detection of the electrophoresed products is accomplished by incorporation of either a fluorescent or radiolabeled tag. Sequencing both strands of 16 kb for a single individual is tedious with current techniques and gives a large amount of unnecessary information, since virtually all of the sequence will be identical to the known sequence. SSCP is less tedious, but still requires sequencing benign polymorphisms. Furthermore SSCP segment length is limited to 150 base pairs, requiring a minimum of 6 PCR reactions per kilobase screened. PTT is the fastest of these techniques but it is incapable of detecting missense mutations, and it also suffers from all of the drawbacks of gel electrophoresis, such as gel preparation, long running times, and low resolution. In addition, PTT will miss some mutations due to artifactual proteins smaller than the wildtype protein that can obscure the signal from a bonafide truncation product. These artifacts can be due to ATG codons within the coding sequence that act as cryptic translation initiation sites. Proteins of this type lack proper amino termini, and hence would lack a peptide tag that one might engineer into the amino termini.

Other techniques such as Allele Specific Oligomer Hybridization (5) and Allele Specific PCR (6) are designed to identify one previously characterized mutation per assay. These techniques are useful when a particular mutation has already been identified and is widespread, but cannot be used for genes where a large number of yet unidentified mutations exist in the human population.

### Objects and Advantages

The objects and advantages of my patent are as follows:
a) to provide a method capable of detecting mutations that qualitatively alter the protein product of any protein encoding gene, using genomic DNA or cDNA as starting material;
b) to provide a method for mutation detection that can take advantage of the speed, high throughput, sensitivity, and accuracy of measuring devices that require purified materials for analysis (such as electrospray mass spectrometry);
c) to improve the protein truncation test by eliminating artifacts caused by cryptic translation initiation.

### Figures

Fig 1 shows the features present in the 5' primer used to PCR amplify a region of coding sequence to be tested.
Fig 2 shows a flow chart of how the process works. The 6 major steps are numbered and are discussed below.

### Description of Figures

### Figure 1

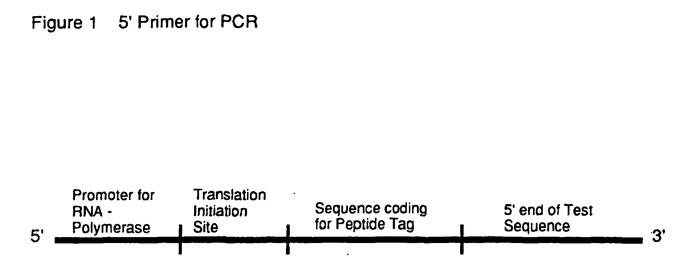

The 5' primer shown in fig 1 is a standard synthetic oligonucleotide without any modifications at either the 5' or 3' ends. The unique features of the oligonucleotide lie in the functional properties of its sequence. Going from the 5' to 3' direction, the following 4 elements are present in the order given:
1) A sequence recognized by an RNA polymerase. An example is the sequence recognized by the viral T7 polymerase TAATACGACTCA-CTATAGGG.
2) A sequence that allows translation initiation of mRNA. An example is the methionine codon placed after the Kozak consensus: AGACCACCATG.
3) An in frame sequence coding for a peptide that can be used for detection or affinity purification (a peptide tag). An example is the sequence GAC TAC AAG GAC GAC GAT GAC AAG, which codes for the FLAG peptide DYKDDDDK. When the FLAG peptide is present at the amino terminus of a protein, it is recognized by the anti-FLAG M2 monoclonal antibody. The M2 antibody can be used in western blotting to detect FLAG containing proteins, or it can be used to affinity purify the protein for further analysis.
4) An in frame sequence complimentary to the 5' end of the coding sequence being tested (the test sequence). This 5' end of the test sequence must be of sufficient length to allow the entire oligomer to hybridize to the test sequence present in the genomic DNA or cDNA sample and to act as a primer for PCR. Usually 20 bases is enough. An example is the in frame sequence GCT TGT GAA TTT TCT GAG AC, which is complimentary to the 5' end of exon 11 of the BRCA1 gene.

### Figure 2

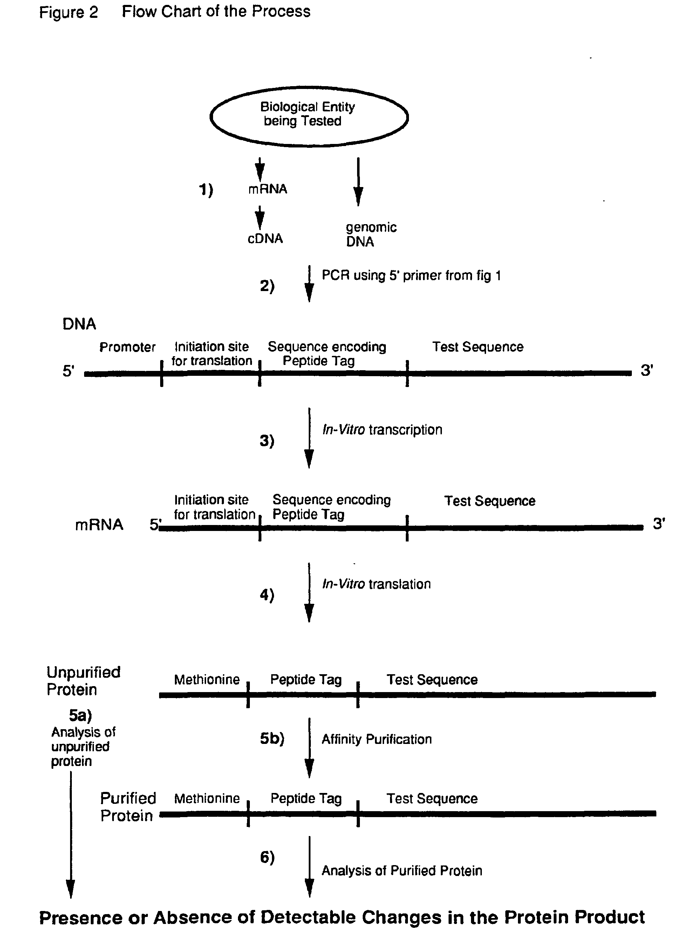

The flow chart in fig 2 shows how the whole process works.
1) Preparation of template for PCR. The PCR reaction must amplify a continuous stretch of coding sequence. The maximum length of coding sequence that can be analyzed per segment is certainly greater than 2 kilobases, thus for genes having many small exons it will be convenient to use cDNA as template. If the mutant message is unstable, this will present a problem, because the absence of the mutant message will result in the lack of mutant cDNA. When the mutant message is unstable in diploid organisms the only template in cDNA derived from a heterozygous carrier would be the wildtype. My experience with the BRCA1 gene has been that the problem of "non-sense mediated mRNA decay" (7), in which message instability is caused by a mutation that generates a premature stop codon, is not really a problem, and I expect that for most genes cDNA will be the template of choice.
   The source of the tissue used to extract RNA must express the gene being tested. Many human genes (if not most) are expressed to some extent in peripheral blood, and can be PCR amplified from cDNA derived from peripheral blood leukocytes. Genomic DNA can be conveniently used as template for the coding sequence of large exons. It can also be used when cDNA is not available, and when mutant message is thought to be unstable. It should be noted that splice site mutations within introns and a subset of those within exons will be not be detected when genomic DNA is used as template.
2) PCR. A continuous region of coding sequence for the gene of interest is amplified by the polymerase chain reaction using the 5' primer described in fig 1, and a 3' primer that need not be in-frame. When cDNA is used as template and the gene is expressed at low levels in the source tissue used to make RNA, nested PCR is often required to get good amplification. An aliquot of the PCR product is used for the next step.
3) *In-Vitro* Transcription. RNA is generated by using the PCR product from step 2 as template in a reaction mixture containing all of the other components required for RNA synthesis. The RNA polymerase used in this step must recognize the promoter sequence at the 5' end of the PCR product.
4) *In-Vitro* Translation. The RNA made in step 3 is then used as template in a cell lysate containing all the reagents required for protein synthesis. Rabbit reticulocytes and wheat germ are common lysate sources. Steps 3 and 4 could be done simultaneously using a coupled transcription/translation system, but this is not essential. Radiolabelled amino acids are included in the lysate if autoradiography will be performed later.
5a) Analysis of Unpurified Protein Using Tag. The unpurified tagged protein made in step 4 can be analyzed directly using ligands specific for the peptide tag. For example, the size of the protein can be determined by western blotting using an antibody directed against the tag. Note that artifactually small proteins resulting from cryptic translation initiation will not be detected by ligands specific for the amino terminal tag.
5b) Affinity Purification. The protein made in step 4 is present in a lysate containing thousands of different proteins, ribosomal RNA, salts, metabolites, lipids, etc. In order to analyze this protein using some of the more sensitive techniques available (such as electrospray mass spectrometry), it is necessary to purify the protein. The peptide tag engineered into the amino-terminus of the protein can be used for this purpose. One way to accomplish the purification is to use a ligand specific for the peptide tag immobilized to a solid surface. For example, if the FLAG peptide is used as the peptide tag, an anti-FLAG antibody could be covalently attached to magnetic beads. After incubation of the lysate with the anti-FLAG beads to allow binding, the beads could be washed, and the test protein could be eluted from the beads in a suitable buffer.
6) Analysis of the Purified Protein. The purified protein can now be analyzed by a variety of techniques that measure the physical, electrical, chemical, or biological properties of the protein. These properties are compared to the properties of the protein encoded by the wildtype sequence and any differences between the test protein and the control indicate that a mutation may be present. The assay used at this stage must be sensitive enough to analyze the amount of material purified from the lysate. For example, 50 microliters of a rabbit reticulocyte lysate may provide 10 picomoles of a 90 kD protein and autoradiography of radiolabelled protein is certainly sensitive enough to detect this amount of protein. Such an approach will be similar to the standard PTT except that the background signals caused by labelled artifactual proteins lacking the tag will be absent. Another method of analysis is electrospray mass spectroscopy which can measure the mass of a protein to within 0.01% when sub-picomole amounts of the protein are available. This level of accuracy should allow for detection of mutant proteins having amino acid substitutions as well as truncations.

### Summary, Ramifications, and Scope

In conclusion, the reader will see that tagged *in-vitro* synthesized proteins have advantages over untagged proteins in terms of how they can be analyzed and the quality of data obtained. Detection of the tag, rather than of all newly synthesized proteins from the lysate, eliminates the background noise caused by artifactual proteins lacking the tag. Furthermore, the tag can be used to purify the protein away from other components of the lysate, including the artifactual proteins lacking the tag, prior to analysis. To date, the exquisite accuracy, sensitivity, and throughput of certain protein analytical techniques have yet to be applied to mutation detection. The ability to easily purify *in-vitro* synthesized proteins may facilitate the use of these techniques in helping to identify disease causing genes in humans. Although I have only used the examples of autoradiography and mass spectroscopy as methods for analyzing the purified proteins, this should not be construed as limiting the scope of the invention, since other techniques may also be used. Rather, the scope of the invention should be determined by the appended claims.

### References

1) Miki Y, Swensen J, Shattuck-Eidens D, et al. A strong candidate for the breast and ovarian cancer susceptibility gene BRCA1. Science 1994;266(5182):66-71.
2) Wooster R, Bignell G, Lancaster J, Swift S, Seal S, et al. Identification of the breast cancer susceptibility gene BRCA2. Nature 1995;378:789-792.
3) Shattuck-Eidens D, McClure M, Simard J, et al. A collaborative survey of 80 mutations in the BRCA1 breast and ovarian cancer susceptibility gene: Implications for presymptomatic testing and screening. Jama 1995;273(7):535-541.
4) Hogervorst FBL, Cornelis RS, Bout M, et al. Rapid detection of BRCA1 mutations by the protein truncation test. Nature genetics 1995;10:208-213.
5) Struewing JP, Abeliovich D, Peretz T, et al. The carrier frequency of the BRCA1 185delAG mutation is approximately 1 percent in Ashkenazi Jewish individuals. Nat Genet 1995;11(2):198-200.
6) Fitzgerald MG, Macdonald DJ, Krainer M, et al. Germline BRCA1 mutations in jewish and non-jewish women with early-onset breast cancer. N Engl J Med 1996;334:143-149.
7) Zhang ZX, Wakamatsu N, Mules EH, Thomas GH, Gravel RA. Impact of premature stop codons on mRNA levels in infantile Sandhoff disease. Hum Mol Genet 1994;3(1):139-45.

## Claims

1. A method for detecting a protein altering mutation in a nucleic acid sequence, comprising:
a) amplifying the sequence using a 3' primer and a 5' primer to create a product, the 5' primer comprising, from its 5' to 3' direction:
i) a RNA polymerase promoter;
ii) a mRNA translation initiation site;
iii) an in frame polynucleotide sequence encoding a peptide tag; and
iv) an in frame polynucleotide sequence that hybridizes to a sequence on the non-coding strand adjacent to the 5' end of the nucleic acid sequence;
b) transcribing *in vitro* the product of step a) to create an RNA product;
c) translating *in vitro* the RNA product of step b) to create a protein comprising said peptide tag;
d) either:
i) purifying the protein of step c) with a ligand specific for the peptide tag; or
ii) detecting the protein of step c) with a ligand specific for the peptide tag;
and
e) analyzing the protein of step d) to determined whether a protein altering mutation is present in the nucleic acid sequence.

2. The method of claim 1, wherein the 3' primer is not in frame.

3. The method of claim 1, wherein the polynucleotide sequence that hybridizes to a sequence on the non-coding strand is 20 bases in length.

4. The method of claim 1, wherein the nucleic acid sequence comprises the BRCA1 or BRCA2 gene.

5. The method of claim 1, wherein the nucleic acid sequence comprises the 5' end of exon 1 of the BRCA1 gene.

6. The method of claim 1, wherein said analyzing of step e) comprises mass spectrometry.

## Patentansprüche

1. Verfahren zum Erkennen einer Proteinveränderungsmutation in einer Nukleinsäuresequenz, aufweisend:
a) Amplifizieren der Sequenz unter Verwendung eines 3'-Primers und eines 5'-Primers, um ein Produkt zu erzeugen, wobei der 5'-Primer von seiner 5'- zur 3'-Richtung hin aufweist:
i) einen RNA-Polymerase-Promotor,
ii) eine mRNA-Translations-Initiierungsstelle,
iii) eine Polynukleotidsequenz im Leserahmen, welche einen Peptid-Tag codiert, und
iv) eine Polynukleotidsequenz im Leserahmen, welche mit einer Sequenz auf dem nicht-codierenden Strang, der dem 5'-Ende der Nukleinsäuresequenz benachbart ist, hybridisiert,
b) in vitro-Transkribieren des Produkts aus Schritt a), um ein RNA-Produkt zu erzeugen,
c) in vitro-Translatieren des RNA-Produkts aus Schritt b), um ein Protein zu erzeugen, welches der Peptid-Tag aufweist,
d) entweder:
i) Reinigen des Proteins aus Schritt c) mit einem Liganden, der spezifisch für den Peptid-Tag ist,
ii) Detektieren des Proteins aus Schritt c) mit einem Liganden, der spezifisch für den Peptid-Tag ist,
und
e) Analysieren des Proteins aus Schritt d), um zu bestimmen, ob eine Proteinveränderungsmutation in der Nukleinsäuresequenz vorhanden ist.

2. Verfahren nach Anspruch 1, wobei der 3'-Primer nicht im Leserahmen ist.

3. Verfahren nach Anspruch 1, wobei die Polynukleotidsequenz, welche mit einer Sequenz auf dem nicht-codierenden Strang hybridisiert, 20 Basen in der Länge beträgt.

4. Verfahren nach Anspruch 1, wobei die Nukleotidsäuresequenz das BRCA1 - oder BRCA2-Gen aufweist.

5. Verfahren nach Anspruch 1, wobei die Nukleotidsequenz das 5'-Ende von Exon 11 des BRCA1-Gens aufweist.

6. Verfahren nach Anspruch 1, wobei die Analyse aus Schritt e) Massenspektrometrie umfasst.

## Revendications

1. Procédé de détection d'une mutation dans une séquence d'acide nucléique altérant une protéine, comportant les étapes consistant à :
a) amplifier la séquence en utilisant une amorce 3' et une amorce 5' pour créer un produit, l'amorce 5' comprenant, dans sa direction de 5' à 3':
i) un promoteur d'ARN polymérase,
ii) un site d'initiation de la traduction d'ARNm,
iii) une séquence polynucléotidique en phase codant une étiquette peptidique, et
iv) une séquence polynucléotidique en phase qui hybride avec une séquence sur le brin non codant adjacente à l'extrémité 5' de la séquence d'acide nucléique,
b) transcrire *in vitro* le produit de l'étape a) pour créer un produit d'ARN,
c) traduire *in vitro* le produit d'ARN de l'étape b) pour créer une protéine comprenant ladite étiquette peptidique,
d) soit :
i) purifier la protéine de l'étape c) avec un ligand spécifique pour l'étiquette peptidique, ou
ii) détecter la protéine de l'étape c) avec un ligand spécifique pour l'étiquette peptidique,
et
e) analyser la protéine de l'étape d) pour déterminer si une mutation altérant la protéine est présente dans la séquence d'acide nucléique.

2. Procédé selon la revendication 1, dans lequel l'amorce 3' n'est pas en phase.

3. Procédé selon la revendication 1, dans lequel la séquence polynucléotidique qui hybride avec une séquence sur le brin non codant est d'une longueur de 20 bases.

4. Procédé selon la revendication 1, dans lequel la séquence d'acide nucléique comprend le gène BRCA 1 ou BRCA 2.

5. Procédé selon la revendication 1, dans lequel la séquence d'acide nucléique comprend l'extrémité 5' de l'exon 11 du gène BRCA 1.

6. Procédé selon la revendication 1, dans lequel ladite analyse de l'étape e) comprend une spectrométrie de masse.
